# EUROPEAN PATENT APPLICATION

(11) **EP 0 627 488 A1**
(43) Date of publication of application: **07.12.1994**
(21) Application number: 94830251.8
(22) Date of filing: 25.05.1994
(51) Int. Cl.: C12N 15/10, C12N 15/62, C12N 7/01, C07K 7/08, C12Q 1/68, C12Q 1/70, G01N 33/53, G01N 33/68

(54) **Method for producing filamentous phages displaying on the surface of the capsid peptides capable of binding biotin, and filamentous phages and peptides thus obtained**

(30) Priority: 01.06.1993 IT RM930361
(71) Applicant: ISTITUTO DI RICERCHE DI BIOLOGIA MOLECOLARE P. ANGELETTI S.P.A., 00040 Pomezia (IT)
(72) Inventor: Laufer, Ralph, I-00144 Roma (IT); Saggio, Isabella, I-04020 Ventotene LT (IT)
(74) Representative: Di Cerbo, Mario

(57) **Abstract**

A method for isolating filamentous phages capable of binding biotin, essentially characterized by the following operations:
- biotinylation of a protein used for the screening of a phage library, in which, on the surface of the capsid, are expressed oligopeptides coded by random sequence oligonucleotide inserts, introduced into a gene coding for a protein of the phage capsid using genetic manipulation techniques;
- screening the above library using the biotinylated protein; and
- isolating the phages capable of binding to biotin.

Figure 1 shows, in the form of a histogram, the binding of biotinylated proteins to phages selected according to the method of the invention and immobilized on plastic.

## Description

The present invention has as its subject a method according to which, using a highly biotinylated protein, it is possible to select phages capable of binding biotin.

As is known, biotin is a small molecule (MW 244) that can be conjugated to a number of macromolecules without altering their size, physical characteristics and biological activity. Biotin has high binding affinity with both avidin and streptavidin. This interaction is used in a number of technological applications for the detection and purification of biotinylated molecules.

However, the use of avidin and/or streptavidin, in spite of the high biotin binding activity of these molecules, shows several disadvantages. The most important of these disadvantages can be summarized as follows. First of all, avidin binds a number of cell types in a non-specific manner, and can also be internalized, which may limit its use in cytochemical methods. Secondly, extremely drastic conditions are required to dissociate the avidin/biotin bond, which limits its use for affinity purification. Anti-biotin antibodies have been produced as alternative reagents; but these can also cause non-specific binding phenomena.

For all the reasons given above, there is in this specific sector a need for a new type of reagent, capable of binding biotin without causing the problems and limits indicated above.

The method according to the present invention allows a new type of reagent to be produced, which offers certain advantages that will be seen from the following.

Subject of the present invention is therefore a method for isolating filamentous phages capable of binding to biotin, essentially characterized by the following operations:
- biotinylation of a protein used for the screening of a phage library, in which, on the surface of the capsid, are expressed oligopeptides coded by random sequence oligonucleotide inserts, introduced into a gene coding for a protein of the phage capsid using genetic manipulation techniques;
- screening the above library using the biotinylated protein; and
- isolating the phages capable of binding to biotin.

The gene coding for the phage capsid, with random sequence oligonucleotidic inserts, can be the gene coding for the protein pVIII or the gene coding for the protein pIII of the phage capsid.

In a specific embodiment of the invention, the random sequence oligonucleotides are inserted in the gene coding for the protein pVIII, using the plasmid pC89 as an expression vector, from the recognition site for the restriction enzyme EcoRI to that for the restriction enzyme BamHI. In this case the oligonucleotides described can have the sequence SEQ ID NO:1.

The present invention is not limited to the method for isolating filamentous phages capable of binding to biotin as described above. It also extends to the phages characterized by the fact that they display, on the surface of the capsid, oligopeptides conferring the phage the ability to bind biotin, and by the fact that they are obtainable using the method described above.

In an embodiment, the peptide inserted in the protein of the phage capsid and conferring the ability to bind biotin, comprises an amino acid sequence selected from SEQ ID NO:2 and SEQ ID NO:3. In this case, the peptide conferring the phage the ability to bind biotin can be inserted in the protein pVIII.

The recombinant protein pVIII, in another embodiment of the invention, can comprise an amino acid sequence selected from SEQ ID NO:4 and SEQ ID NO:5.

In a further embodiment of the invention the mature recombinant protein pVIII can comprise, between the amino acid No. 3 (Gly) and the amino acid No. 5 (Asp), an amino acid sequence selected from SEQ ID NO:6 and SEQ ID NO:7. In this case the recombinant protein can comprise an amino acid sequence chosen from the group comprising SEQ ID NO:8, SEQ ID NO:9 and SEQ ID NO:10. The amino acid sequence comprising the sequence SEQ ID NO:8 can be the amino acid sequence SEQ ID NO:11.

The invention also extends to the peptides capable of binding biotin which comprise an amino acid sequence selected from the group comprising SEQ ID NO:3, SEQ ID NO:4, SEQ ID NO:5, SEQ ID NO:6 and SEQ ID NO: 7.

The peptides capable of binding biotin that include the sequences SEQ ID NO:6 and SEQ ID NO:7 can include a sequence chosen from the group comprising the amino acid sequences SEQ ID NO:8, SEQ ID NO:9 and SEQ ID NO:10.

In a special embodiment, the peptide capable of binding biotin comprising the sequence SEQ ID NO:8 according to the present invention, may consist in the amino acid sequence SEQ ID NO:11.

The filamentous phages according to the present invention may be used for detection of biotinylated molecules in methods chosen, for example, from the group comprising ELISA, Western, Southern and Northern blotting, histochemistry and receptor binding assays.

It is also possible to use filamentous phages according to the present invention for the purification of biotinylated molecules.

Like the filamentous phages according to the present invention, the peptides according to the present invention can also be used for detection of biotinylated molecules in methods chosen, for example, from the group comprising ELISA, Western, Southern and Northern blotting, histochemistry and receptor binding assays, or for the purification of biotinylated molecules.

So far a description of a general nature has been given of the subjects of the present invention. With the aid of the following examples a more detailed description will now be given of specific embodiments of the invention, with the aim of giving a better understanding of the objects, characteristics, advantages and way of application thereof.

Figure 1, in the form of a histogram, shows the binding of biotinylated proteins to phages selected according to the present invention and immobilized on plastic.

Figure 2, in the form of a histogram, shows the binding of phages selected according to the invention to biotinylated proteins immobilized on plastic.

Figure 3 shows, in the form of a histogram, the identification of proteins immobilized on plastic by means of a biotinylated ligand.

Figure 4 shows curve relating to inhibition of binding of streptavidin, marked with ¹²⁵I, to biotinylated bovine serum albumin (BSA), immobilized on plastic, with increasing amounts of the peptide with sequence SEQ ID NO:11.

### DEPOSITS

Xl1-Blue bacteria - transformed using the plasmid pC89 which contains, from the recognition site for the restriction enzyme EcoRI to that for the restriction enzyme BamHI, the nucleotidic sequence coding for the amino acidic sequence SEQ ID NO:5 - were deposited on 26/5/93 with The National Collections of Industrial and Marine Bacteria Ltd. (NCIMB), Aberdeen, Scotland, UK, under access number NCIMB 40561.

### Example 1

### Process for isolation of phages capable of binding biotin

1) Biotinylation of a protein used for screening of a phage library.
   A cell line producing mAb 35, a rat monoclonal antibody directed against the acetylcholine receptor, was obtained from the American Type Culture Collection (Rockville, Maryland, USA). The antibody was precipitated from serum-free culture medium by addition of an equal volume of a solution saturated with ammonium sulphate. After incubation for 4 hours at 4°C, the precipitate was recovered by centrifugation, resuspended in PBS and exhaustively dialyzed against 10 mM Tris-HCl, pH 8.0.
   The biotinylation was performed by a modification of the method of Parmley and Smith (1988) [Gene 73, 305-318], using a ratio of 4 times more reagent with respect to the protein. The mAB 35 (6 nmoles) was incubated with sulphosuccinimidyl-6-(biotinamido)hexanoate (NHS-LS-biotin Pierce, 360 nmoles) in 0.1 sodium bicarbonate, pH 8.2, for 2 hours at room temperature. Ethanolamine 1 M, pH 9 (0.5 ml) was added and incubation was continued for a further 2 hours at room temperature. Following addition of BSA to a final concentration of 1 mg/ml, the product was concentrated on a "Centricon 30 ultrafilter" (Amicon), and the retentate was washed three times with 50 mM Tris-HCl, 150 mM NaCl, pH 7.4 (TBS).
2) Screening of a peptide library expressed on phage with biotinylated mAB 35.

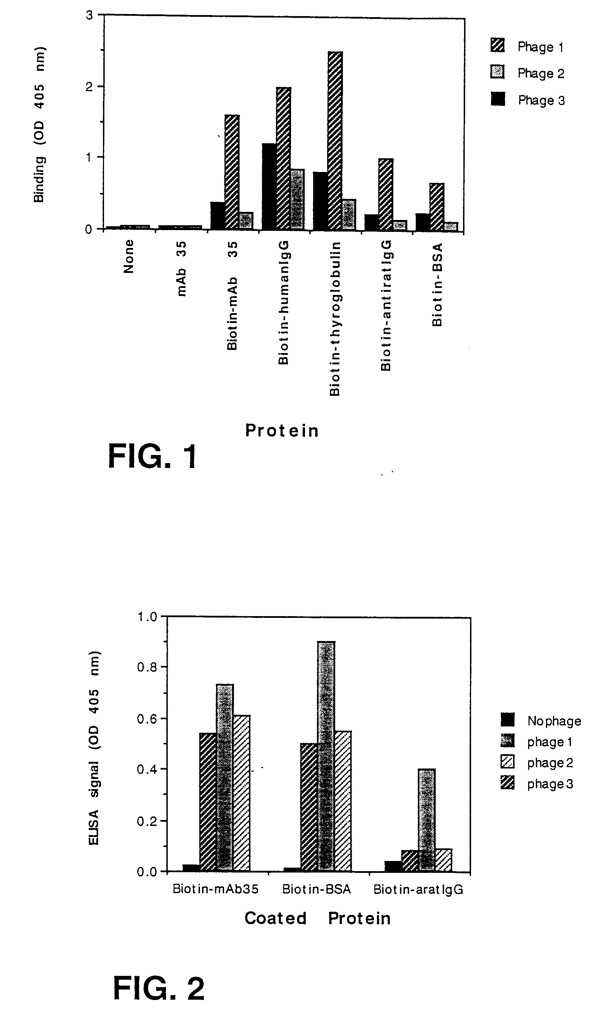

The phage library was constructed [Luzzago et al. (1993) Gene], using a modified pC89 plasmidic expression vector [Felici et al. (1991) J. Mol. Biol. 222, 301], containing the gene coding for the main coat protein M13. The recombinant protein pVIII expressed on the surface of the phage contains nonapeptide inserts flanked by two cysteine residues. Three phages capable of binding biotinylated mAB 35 were isolated with three cycles of "biopanning" (Parmley and Smith, work already cited), using concentrations of biotinylated antibody of 1000, 10 and 1 nM, respectively. Amplification, propagation and purification of the phages were performed as described by Felici et al. (work already cited). The above mentioned phages do not bind the non-biotinylated version of the antibody (see example 2, figure 1). They have the following peptide inserts within the protein pVIII:
- SEQ ID NO: 8: (phage number 1);
- SEQ ID NO: 9: (phage number 2); and
- SEQ ID NO:10: (phage number 3).

The sequences indicated are coded by nucleotidic sequences inserted in the plasmid vector of expression pC89, containing the gene for pVIII, from the recognition site for the restriction enzyme EcoRI to that for BamHI.

### Example 2

### Use of the selected Phages immobilized on a solid phase to bind biotinylated molecules

Polystyrene 96-well plates (Probind, Falcon) were coated with 4 x 10¹⁰ CsCl-purified phage particles, diluted in 35 µl of TBS and left overnight at 4°C. The wells were then washed with the following solution: TBS with 5% non-fat dry milk and 0.05% Tween 20 (solution A), then incubated with solution A for 90' at room temperature and washed three times with TBS containing 0.5% Tween 20 (v/v) (TBS-Tween). The antibodies or the biotinylated proteins to be examined, diluted in 50 µl of PBS 0.5% Triton X-100 (v/v), BSA 1 mg/ml, were added to the wells and left to incubate overnight at 4°C. After washing 5 times with TBS-Tween, the wells were incubated for 4 hours at 4°C with 50 µl of TBS containing BSA 1 mg/ml and either avidin conjugated with alkaline phosphatase (Jackson Immunoresearch Labs), 1:1000 (v/v), for detection of biotinylated proteins, or alkaline phosphatase conjugated antibody against rag IgG (Calbiochem) 1:1000 (v/v) for detection of mAb 35. The wells were then washed 5 times with TBS-Tween and developped at 37°C with p-nitrophenylphosphate 1 mg/ml in 1M diethanolamine hydrochloride, pH 9.8. Absorbance at 405 nm was determined using a Biorad microplate reader.

The three phages according to example 1 are capable of binding mAb 35 (1nM) only in its biotinylated form. They are also capable of binding biotinylated thyroglobulin (1nM), biotinylated bovine seric albumin (10 nM), and human biotinylated IgG (1 nM), as illustrated in figure 1.

### Example 3

### Use of the selected phages to detect biotinylated molecules immobilized on a solid phase

Polystyrene 96-well plates were coated with biotinylated proteins diluted in 50 mM sodium bicarbonate, pH 9.5. Following washing and incubation with solution A, according to example 2, the wells were incubated overnight at 4°C with the phages diluted in 50 µl PBS, 0.5% Triton X-100 (v/v), BSA 1 mg/ml. The wells were then washed 5 times with TBS-Tween, and incubated for 2 hours at 4°C with 100 µl of polyclonal antiserum against phage M13, diluted in solution A. After washing 5 times, as above, the wells were incubated for 2 hours at 4°C with alkaline phosphatase conjugated goat antibodies against rabbit IgC (Calbiochem), diluted in solution A. Washing and development of the wells are then performed as described in example 2.

The three phages binding biotin (numbers 1, 2 and 3) were used to detect: biotinylated mAb 35 (10 ng), biotinylated human IgG (5 ng), biotinylated bovine thyroglobulin (5 ng), a goat antibody against biotinylated rat IgG (100 ng) and biotinylated BSA (100 ng), as shown in figure 2. The above mentioned phages do not bind the same proteins in their non-biotinylated form.

### Example 4

### Use of the selected phages for detection of proteins immobilized on a solid Phase by means of a biotinylated ligand

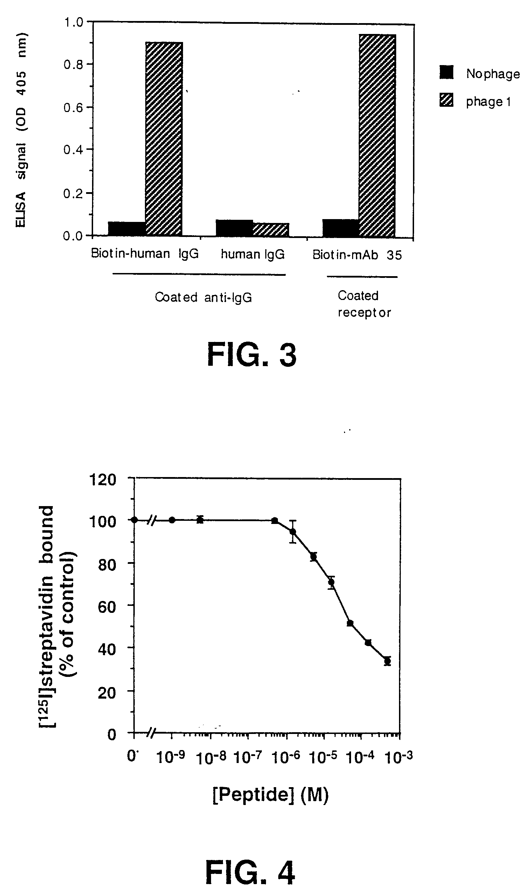

Polystyrene 96-well plates were coated, as described in example 3, with 130 ng goat antiserum against human IgG (Calbiochem) or with 20 ng affinity-purified acetyl-choline receptor (donated by the Unite de Neurobiologie Moleculaire, Institut Pasteur, Paris). Following washing and incubation in solution A, according to example 3, the wells containing human anti-IgG were incubated for 2 hours at room temperature with 1 nM biotinylated or non-biotinylated human IgG; those containing the receptor, on the other hand, were incubated with 1nM biotinylated mAb 35. After washing five times with TBS-Tween, the wells were incubated for 2 hours, at room temperature, with phage No. 1 (according to example 1) at a concentration of 2 x 10¹² particles/ml solution A. Washing and detection of the phage were performed according to example 3. As shown in figure 3, phage number 1 can be used for detection of protein immobilized on a solid phase, using a biotinylated ligand as an intermediary. Phage No. 1 is not capable of detecting the immobilized phages when a non-biotinylated ligand of the same type is used.

### Example 5

### Demonstration of the ability of the peptide with sequence SEQ ID NO:11 to bind biotin

Polystyrene 96-well plates were coated with 20 ng of biotinylated BSA in 50 mM sodium bicarbonate, pH 9.6. The wells were washed 5 times with PBS and incubated for 1 hour at room temperature with 3 mg/ml BSA in PBS. These were then preincubated for 1 hour at room temperature with 40 µl of 0.2 M Hepes, 2 M NaCl, BSA 1 mg/ml, pH 7, in the presence or in the absence of the peptide with sequence SEQ ID NO:8. The wells were then additioned with 10ul of the same solution containing 8 fmoles (40000 cpm) of [¹²⁵I]streptavidine (Amersham); incubation took place for one night at 4°C. After washing 5 times with PBS, the bound [¹²⁵I]streptavidin was solubilized by addition of 0.2 N NaOH, 1% SDS and then counted using a gamma radiation counter.

As indicated in figure 4, the peptide in question is capable of inhibiting binding of the radio-labelled streptavidin to the biotinylated compound with an IC₅₀ of 50 µM.

## Claims

1. A method for isolating filamentous phages capable of binding biotin, essentially characterized by the following operations:
- biotinylation of a protein used for the screening of a phage library, in which, on the surface of the capsid, are expressed oligopeptides coded by random sequence oligonucleotide inserts, introduced into a gene coding for a protein of phage capsid using genetic manipulation techniques;
- screening the above library using the biotinylated protein; and
- isolating the phages capable of binding to biotin.

2. A method for isolating filamentous phages capable of binding to biotin according to claim 1, in which the gene coding for the phage capsid, with random sequence oligonucleotidic inserts, is the gene coding for the protein pVIII or the gene coding for the protein pIII of the phage capsid.

3. A method for isolating filamentous phages capable of binding to biotin according to claim 1 or 2, in which the random sequence oligonucleotides are inserted in the gene coding for the protein pVIII, using the plasmid pC89 as a vector of expression, from the recognition site for the restriction enzyme EcoRI to that of restriction enzyme BamHI.

4. A method for isolating filamentous phages capable of binding to biotin according to claim 3, in which the oligonucleotides inserted have the sequence SEQ ID NO: 1.

5. Filamentous phages, characterized by the fact that they display, on the surface of the capsid, oligopeptides giving the phage the ability to bind biotin, and by the fact that they are obtainable using the method according to any one of claims 1 to 4.

6. Filamentous phages according to claim 5, in which the peptide inserted in the phage capsid protein, which confers the ability to bind biotin, comprises an amino acid sequence selected from SEQ ID NO:2 and SEQ ID NO:3.

7. Filamentous phages according to claim 6 in which the peptide giving the phage the ability to bind biotin is inserted in the protein pVIII.

8. Filamentous phages according to claim 7, in which the recombinant protein pVIII comprises an amino acid sequence selected from SEQ ID NO:4 and SEQ ID NO:5.

9. Filamentous phages according to claim 8, in which the mature recombinant protein pVIII comprises, between amino acid No. 3 (Gly) and amino acid No. 5 (Asp), an amino acid sequence selected from SEQ ID NO:6 and SEQ ID NO:7.

10. Filamentous phages according to claim 9, in which the recombinant protein comprises an amino acid sequence chosen from the group comprising SEQ ID NO:8, SEQ ID NO:9 and SEQ ID NO:10.

11. Peptides capable of binding biotin, comprising an amino acid sequence selected from SEQ ID NO:2 and SEQ ID NO:3.

12. Peptides according to claim 11, comprising an amino acid sequence selected from SEQ ID NO:4 and SEQ ID NO:5.

13. Peptides according to claim 12, comprising an amino acid sequence selected from SEQ ID NO:6 and SEQ ID NO: 7.

14. Peptides-according to claim 13, comprising a sequence chosen from the group comprising the amino acid sequences SEQ ID NO:8, SEQ ID NO:9 and SEQ ID NO:10.

15. Peptide according to claim 14, in which the amino acid sequence comprising the sequence SEQ ID NO:8 consists of the amino acid sequence SEQ ID NO:11.

16. Use of filamentous phages according to claims 5 to 10 for detection of biotinylate molecules in methods chosen, for example, from the group comprising ELISA, Western, Southern and Northern blotting, histochemistry and receptor binding assays.

17. Use of filamentous phages according to claims 5 to 10, for purification of biotinylated molecules.

18. Use of peptides according to claims 11 to 15 for detection of biotinylated molecules in methods chosen, for example, from the group comprising ELISA, Western, Southern and Northern blotting, histochemistry and receptor binding assays.

19. Use of peptides according to claims 11 to 15 for purification of biotinylated molecules.


SEQUENCE LISTING
(1) GENERAL INFORMATION
(i) APPLICANT: ISTITUTO DI RICERCHE DI BIOLOGIA
MOLECOLARE P. ANGELETTI S.p.A.
(ii) TITLE OF INVENTION: Method for producing
filamentous phages diplaying on the surface of the
capsid peptides capable of binding biotin, and
filamentous phages and peptides thus obtainable
(iii) NUMBER OF SEQUENCES: 11
(iv) COMPUTER READABLE FORM:
(A) MEDIUM TYPE: Floppy disk 3.5" 1.44 MBYTES
(B) COMPUTER: IBM PC compatible
(C) OPERATING SYSTEM: PC-DOS/MS-DOS Rev. 5.0
(D) SOFTWARE: Microsoft Wordstar 4.0
(v) CORRESPONDENCE ADDRESS:
(A) ADDRESSEE: Società Italiana Brevetti
(B) STREET: Piazza di Pietra, 39
(C) CITY: Rome
(D) COUNTRY: Italy
(E) POSTAL CODE: I-00186
(viii) ATTORNEY INFORMATION
(A) NAME: DI CERBO, Mario (Dr.)
(C) REFERENCE: RM/090075/MDC
(ix) TELECOMMUNICATION INFORMATION
(A) TELEPHONE: 06/6785941
(B) TELEFAX: 06/6794692
(C) TELEX: 612287 ROPAT
(2) INFORMATION FOR SEQ ID NO: 1:
(i) SEQUENCE CHARACTERISTICS
(A) LENGTH: 45 base pairs
(B) TYPE: nucleic acid
(C) STRANDEDNESS: single
(D) TOPOLOGY: linear
(ii) MOLECULE TYPE: DNA
(iii) HYPOTHETICAL: no
(iv) ANTISENSE: no
(v) FRAGMENT TYPE: internal

(vii) IMMEDIATE SOURCE
(A) SYNTHESIS: synthesis from nucleotides
(ix) FEATURE
(A) NAME: oligonucleotide
(xi) SEQUENCE DESCRIPTION: SEQ ID NO: 1:
GAATTCTGCN NNNNNNNNNN NNNNNNNNNN NNNNNNTGCG GGGAT 45
(3) INFORMATION FOR SEQ ID NO : 2:
(i) SEQUENCE CHARACTERISTICS
(A) LENGTH: 6 amino acids
(B) TYPE: amino acid
(C) STRANDEDNESS: single
(D) TOPOLOGY: linear
(ii) MOLECULE TYPE: recombinant protein
(iii) HYPOTHETICAL: yes
(v) FRAGMENT TYPE: internal
(vii) IMMEDIATE SOURCE
(A) LIBRARY: of recombinant peptides on phage
(B) CLONE: phagic
(ix) FEATURE
(A) NAME: peptide
(B) IDENTIFICATION METHOD: selection with
biotinylated protein
(D) OTHER INFORMATION: Xaa is any amino acid
that does not prejudice the ability to
bind biotin
(xi) SEQUENCE DESCRIPTION: SEQ ID NO : 2:
Trp Xaa Pro Pro Phe Lys
1               5
(4) INFORMATION FOR SEQ ID NO : 3:
(i) SEQUENCE CHARACTERISTICS
(A) LENGTH: 6 amino acids
(B) TYPE: amino acid
(C) STRANDEDNESS: single
(D) TOPOLOGY: linear
(ii) MOLECULE TYPE: recombinant protein
(iii) HYPOTHETICAL: yes
(v) FRAGMENT TYPE: internal

(vii) IMMEDIATE SOURCE
(A) LIBRARY: of recombinant peptides on phage
(B) CLONE: phagic
(ix) FEATURE
(A) NAME: peptide
(B) IDENTIFICATION METHOD: selection with
biotinylated protein
(D) OTHER INFORMATION: Xaa is any amino acid
that does not prejudice the ability to
bind biotin
(xi) SEQUENCE DESCRIPTION: SEQ ID NO : 3:
Trp Xaa Pro Pro Phe Arg
1               5
(5) INFORMATION FOR SEQ ID NO : 4:
(i) SEQUENCE CHARACTERISTICS
(A) LENGTH: 11 amino acids
(B) TYPE: amino acid
(C) STRANDEDNESS: single
(D) TOPOLOGY: linear
(ii) MOLECULE TYPE: recombinant protein
(iii) HYPOTHETICAL: yes
(v) FRAGMENT TYPE: internal
(vii) IMMEDIATE SOURCE
(A) LIBRARY: of recombinant peptides on phage
(B) CLONE: phagic
(ix) FEATURE
(A) NAME: peptide
(B) IDENTIFICATION METHOD: selection with
biotinylated protein
(D) OTHER INFORMATION: Xaa is any amino acid
that does not prejudice the ability to
bind biotin
(xi) SEQUENCE DESCRIPTION: SEQ ID NO : 4:
Cys Xaa Trp Xaa Pro Pro Phe Lys Xaa Xaa Cys
1               5                   10
(6) INFORMATION FOR SEQ ID NO : 5:
(i) SEQUENCE CHARACTERISTICS

(A) LENGTH: 11 amino acids
(B) TYPE: amino acid
(C) STRANDEDNESS: single
(D) TOPOLOGY: linear
(ii) MOLECULE TYPE: recombinant protein
(iii) HYPOTHETICAL: yes
(v) FRAGMENT TYPE: internal
(vii) IMMEDIATE SOURCE
(A) LIBRARY: of recombinant peptides on phage
(B) CLONE: phagic
(ix) FEATURE
(A) NAME: peptide
(B) IDENTIFICATION METHOD: selection with
biotinylated protein
(D) OTHER INFORMATION: Xaa is any amino acid
that does not prejudice the ability to
bind biotin
(xi) SEQUENCE DESCRIPTION: SEQ ID NO : 5
Cys Xaa Trp Xaa Pro Pro Phe Arg Xaa Xaa Cys
1               5                   10
(7) INFORMATION FOR SEQ ID NO : 6:
(i) SEQUENCE CHARACTERISTICS
(A) LENGTH: 14 amino acids
(B) TYPE: amino acid
(C) STRANDEDNESS: single
(D) TOPOLOGY: linear
(ii) MOLECULE TYPE: recombinant protein
(iii) HYPOTHETICAL: yes
(v) FRAGMENT TYPE: internal
(vii) IMMEDIATE SOURCE
(A) LIBRARY: of recombinant peptides on phage
(B) CLONE: phagic
(ix) FEATURE
(A) NAME: peptide
(B) IDENTIFICATION METHOD: selection with
biotinylated protein
(D) OTHER INFORMATION: Xaa is any amino acid

that does not prejudice the ability to
bind biotin
(xi) SEQUENCE DESCRIPTION: SEQ ID NO : 6
Glu Phe Cys Xaa Trp Xaa Pro Pro Phe Lys Xaa Xaa Cys Gly
1               5                   10
(8) INFORMATION FOR SEQ ID NO : 7:
(i) SEQUENCE CHARACTERISTICS
(A) LENGTH: 14 amino acids
(B) TYPE: amino acid
(C) STRANDEDNESS: single
(D) TOPOLOGY: linear
(ii) MOLECULE TYPE: recombinant protein
(iii) HYPOTHETICAL: yes
(v) FRAGMENT TYPE: internal
(vii) IMMEDIATE SOURCE
(A) LIBRARY: of recombinant peptides on phage
(B) CLONE: phagic
(ix) FEATURE
(A) NAME: peptide
(B) IDENTIFICATION METHOD: selection with
biotinylated protein
(D) OTHER INFORMATION: Xaa is any amino acid
that does not prejudice the ability to
bind biotin
(xi) SEQUENCE DESCRIPTION: SEQ ID NO : 7
Glu Phe Cys Xaa Trp Xaa Pro Pro Phe Arg Xaa Xaa Cys Gly
1               5                   10
(9) INFORMATION FOR SEQ ID NO : 8:
(i) SEQUENCE CHARACTERISTICS
(A) LENGTH: 14 amino acids
(B) TYPE: amino acid
(C) STRANDEDNESS: single
(D) TOPOLOGY: linear
(ii) MOLECULE TYPE: recombinant protein
(iii) HYPOTHETICAL: yes
(v) FRAGMENT TYPE: internal
(vii) IMMEDIATE SOURCE

(A) LIBRARY: of recombinant peptides on phage
(B) CLONE: phagic
(ix) FEATURE
(A) NAME: peptide
(B) IDENTIFICATION METHOD: selection with
biotinylated protein
(xi) SEQUENCE DESCRIPTION: SEQ ID NO : 8:
Glu Phe Cys Ser Trp Ala Pro Pro Phe Lys Ala Ser Cys Gly
1               5                   10
(10) INFORMATION FOR SEQ ID NO : 9:
(i) SEQUENCE CHARACTERISTICS
(A) LENGTH: 14 amino acids
(B) TYPE: amino acid
(C) STRANDEDNESS: single
(D) TOPOLOGY: linear
(ii) MOLECULE TYPE: recombinant protein
(iii) HYPOTHETICAL: yes
(v) FRAGMENT TYPE: internal
(vii) IMMEDIATE SOURCE
(A) LIBRARY: of recombinant peptides on phage
(B) CLONE: phagic
(ix) FEATURE
(A) NAME: peptide
(B) IDENTIFICATION METHOD: selection with
biotinylated protein
(xi) SEQUENCE DESCRIPTION: SEQ ID NO : 9:
Glu Phe Cys Asn Trp Thr Pro Pro Phe Lys Thr Arg Cys Gly
1               5                   10
(11) INFORMATION FOR SEQ ID NO : 10:
(i) SEQUENCE CHARACTERISTICS
(A) LENGTH: 14 amino acids
(B) TYPE: amino acid
(C) STRANDEDNESS: single
(D) TOPOLOGY: linear
(ii) MOLECULE TYPE: recombinant protein
(iii) HYPOTHETICAL: yes
(v) FRAGMENT TYPE: internal

(vii) IMMEDIATE SOURCE
(A) LIBRARY: of recombinant peptides on phage
(B) CLONE: phagic
(ix) FEATURE
(A) NAME: peptide
(B) IDENTIFICATION METHOD: selection with
biotinylated protein
(xi) SEQUENCE DESCRIPTION: SEQ ID NO : 10
Glu Phe Cys Ser Trp Arg Pro Pro Phe Arg Ala Val Cys Gly
1               5                   10
(12) INFORMATION FOR SEQ ID NO : 11:
(i) SEQUENCE CHARACTERISTICS
(A) LENGTH: 21 amino acids
(B) TYPE: amino acid
(C) STRANDEDNESS: single
(D) TOPOLOGY: linear
(ii) MOLECULE TYPE: synthetic peptide
(iii) HYPOTHETICAL: yes
(v) FRAGMENT TYPE: internal
(vii) IMMEDIATE SOURCE
(A) LIBRARY: of recombinant peptides on phage
(ix) FEATURE
(A) NAME: peptide
(B) IDENTIFICATION METHOD:
(xi) SEQUENCE DESCRIPTION: SEQ ID NO : 11:
Ala Glu Gly Glu Phe Cys Ser Trp Ala Pro Pro Phe Lys Ala Ser Cys
1               5                   10                  15
Gly Asp Pro Ala Lys
            20